# FASCICULE DE BREVET EUROPEEN

(11) **EP 3 220 803 B1**
(45) Date de publication et mention de la délivrance du brevet: **04.12.2019**
(21) Numéro de dépôt: 15804902.3
(22) Date de dépôt: 19.11.2015
(51) Int. Cl.: A61B 5/00, A61B 5/0476, A61B 5/026, A61B 5/0478, A61B 5/1455, G06F 3/01

(54) **DISPOSITIF POUR LA MESURE DES SIGNAUX DE L'ACTIVITÉ CÉRÉBRALE D'UN INDIVIDU**
VORRICHTUNG ZUM MESSEN DER HIRNAKTIVITÄTSSIGNALE EINES INDIVIDUUMS
DEVICE FOR MEASURING THE BRAIN ACTIVITY SIGNALS OF AN INDIVIDUAL

(30) Priorité: 21.11.2014 FR 1461283
(43) Date de publication de la demande: 27.09.2017
(73) Titulaire: Centre Hospitalier Universitaire, 80080 Amiens (FR); Université Amiens Picardie Jules Verne, 80090 Amiens (FR); INSERM (Institut National de la Santé et de la Recherche Médicale), 75013 Paris (FR); Etablissements Malterre Sarl, 80110 Moreuil (FR)
(72) Inventeur: WALLOIS, Fabrice, 80000 Amiens (FR); MAHMOUDZADEH, Mahdi, 80036 Amiens (FR); GREBE, Reinhard, 80680 Hebecourt (FR); MALTERRE, Laurent, 80110 Moreuil (FR); SAFAIE, Javad, 80090 Amiens (FR)
(74) Mandataire: Plasseraud IP
(86) Numéro de dépôt international: PCT/FR2015/053132
(87) Numéro de publication internationale: WO 2016/079436

(56) Documents cités:
- WO-A1-2012/156643
- WO-A1-2012/156643
- WO-A1-2014/169241
- US-A- 3 998 213
- US-A1- 2005 107 716
- US-A1- 2009 088 619
- US-A1- 2014 276 183
- US-B1- 8 019 402
- US-B1- 8 019 402

## Description

La présente invention est relative à un dispositif pour la mesure des signaux de l'activité cérébrale, apte à être positionné sur la tête d'un individu.

Le dispositif trouvera une application particulière pour mesurer, d'une part, par électroencéphalographie l'activité électrique du cerveau, et d'autre part, par spectroscopie dans le proche infrarouge, des données relatives aux conditions hydrodynamiques de la circulation dans le cerveau, tel que l'oxygénation tissulaire.

Il est connu des dispositifs d'électroencéphalographie à électrodes qui permettent l'acquisition d'un électroencéphalogramme, c'est-à-dire l'enregistrement sous la forme d'un signal de l'activité électrique du cerveau. Ces dispositifs utilisent des capteurs constituant des électrodes électriques qui sont disposés sur la tête d'individus à diagnostiquer ou à explorer grâce à des casques ou bonnets, dénommés usuellement « Casque d'ElectroEncéphaloGraphie » (ou casque EEG). De tels bonnets en tissus ont été commercialisés sous la marque « Easycap ».

Les casques en tissu présentent l'inconvénient de ne pas pouvoir manipuler aisément les cheveux de l'individu qui perturbent les signaux d'acquisition de spectroscopie dans le proche infrarouge. En effet, les cheveux doivent être écartés du champ de mesure.

Par ailleurs, la spectroscopie proche de l'infrarouge (SPIR), dénommée également "NIRS" pour Near-infrared spectroscopy (en anglais) dans des longueurs d'ondes comprises entre 600 et 900 nm, permet de rendre compte de l'activité hémodynamique du cerveau sous forme d'un signal, les capteurs utilisés étant des détecteurs (fibres optiques, photodiodes) ou émetteurs (Fibres optiques LED) optiques de photons, que l'on appelle également " optodes ".

Bien souvent, ces dispositifs sont équipés d'électrodes seulement, et conçus seulement pour réaliser un électroencéphalogramme, ou alternativement équipés d'émetteurs et de détecteurs optiques et conçus pour réaliser une spectroscopie proche de l'infrarouge. Autrement dit ces dispositifs dédiés ne permettent pas la mesure simultanée de signaux par électroencéphalographie et par spectroscopie dans le proche infrarouge.

Les casques en tissu conçus pour les électrodes sont trop souples pour autoriser correctement l'agencement par rapport à la surface de la tête des optodes dont les supports trop lourds induisent une déformation du tissu. Ces casques en tissu trop lâches empêchent de conserver une disposition des optodes strictement perpendiculaire au crâne de l'individu, et cela durant toute la durée de l'enregistrement. Aussi, de tels casques souples ne permettent pas d'obtenir une résolution spatiale précise et une mesure fiable des signaux des activités électriques et hémodynamique d'origine cérébrale.

L'état de la technique connait toutefois du document FR 2 899 089 un dispositif de capteurs de signaux comprenant une électrode et une optode disposées sur un même support et concentriquement par rapport à un axe destiné à être sensiblement perpendiculaire à la tête. Un tel dispositif permet avantageusement de cibler précisément une même zone d'intérêt sur la tête de l'individu et de manière à permettre la mesure simultanée de signaux par électroencéphalographie et par spectroscopie dans le proche infrarouge. Un tel dispositif capteur EEG/NIRS présente toutefois l'inconvénient d'un prix de revient non compétitif, par rapport à deux capteurs structurellement indépendants, respectivement une éléctrode et une optode.

On connaît des casques plus rigides qui allient le port des électrodes et celui des optodes, dits casques modulaires, tels que ceux commercialisés sous la marque Shimadzu®. Ces casques en matière plastique relativement rigide sont du type polypropylène et couvrent l'ensemble de la tête en étant doté d'une pluralité d'orifices distincts selon le type de capteur à recevoir.

Toutefois, l'agencement sur la tête d'une personne des dispositifs décrits ci-dessus n'est pas satisfaisant au regard de la qualité des contacts des dispositifs électriques et optiques du fait des courbures de la tête, des cheveux et du manque de flexibilité du dispositif, en particulier pour les jeunes enfants et les nouveaux nés. On connaît par ailleurs des structures qui ne sont pas des bonnets et qui présentent des bandes ou sangles de latex ou de caoutchouc qui relient entres elles des nœuds formant des supports de capteurs ou électrodes. La structure faite ainsi sous forme de réseau ménage des évidements pour le passage des cheveux. On peut citer par exemple les demandes de brevet FR 2 627 975, US 2010/274153, EP 0 541 393 et WO 99/22642.

Cependant, les emplacements des nœuds sont pré-établis et ne permettent pas toujours le positionnement idoine des capteurs et des électrodes selon la tête de l'individu. En outre, pour certaines structures, les sangles élastiques favorisent la sudation et peuvent provoquer une pression d'application trop importante sur la tête, en particulier d'un nourrisson.

On connaît encore de la Société NR Sign Inc. un casque « EEG » (http://www.nrsign.com/eeg-cap/) dont la structure de casque comprend deux armatures reliées par une mentonnière. Ces deux armatures sont destinées à être disposées, de part et d'autre de la tête, respectivement au moins localement en dessous des oreilles. Un ensemble de fils élastiques en silicone, relient les deux armatures en contournant la tête par le dessus, les fils s'étendant latéralement à la tête, et de manière espacée les uns des autres. Une fois mis en tension entre les deux armatures, chaque fil en silicone permet de maintenir un support d'électrode, pincé élastiquement entre la tête de l'individu et le fil silicone, le fil silicone alors en simple appui sur le support d'électrode.

Un tel dispositif présente pour avantage de permettre un réglage précis (continu) du support d'électrode le long du fil silicone.

En revanche, et selon les constatations des inventeurs, un tel casque présente des inconvénients majeurs, et en particulier :
- la conception et l'encombrement des supports de capteur n'autorisent que le positionnement d'un faible nombre d'électrodes EEG sur la tête d'un individu ; un tel dispositif ne permet en aucun cas les positionnements simultanés de capteurs d'électrodes et de capteurs d'optodes, structurellement indépendants, et selon une concentration permettant de cibler des mêmes zones d'intérêt,
- la mise en place d'un tel casque, en particulier le réglage des positions des supports de capteur le long des fils silicones doit obligatoirement se faire directement sur la tête de l'individu : autrement dit, il n'est pas possible de prédisposer les supports de capteur sur une tête de mannequin en ce que, une fois le casque retiré du mannequin, les réglages de positionnement sont perdus.

On connaît encore du document WO2012/156643 un dispositif pour la mesure de signaux de l'activité cérébrale destiné à être apposé sur la tête d'un individu, et permettant de mesurer simultanément des signaux par électroencéphalographie et par spectroscopie dans le proche infrarouge, le dispositif présentant des pièces pour accueillir des électrodes, d'une part, et d'autres pièces pour accueillir des détecteurs et émetteurs optiques (optode), d'autre part.

La structure de ce dispositif comprend une chaîne centrale et des chaînes latérales, chacune résultant de l'assemblage de maillons constitutifs, en articulation les uns par rapport aux autres. Certains des maillons comprennent les pièces adaptées au support des électrodes, d'autres maillons des pièces au support d'optodes.

Selon les constatations des inventeurs, une telle structure de dispositif présente les inconvénients suivants :
- les chaînes (centrales ou latérales), résultant de l'assemblage de maillons rigides sont à l'origine de douleurs pour l'individu, en ce que les chaînes appuient sur la tête en une pluralité de contacts « durs »,
- une telle structure à maillons permet certes un réglage des positions des capteurs (électrodes ou optodes) en différentes positions de montage possibles sur les maillons. Toutefois, il s'agit seulement d'un réglage de position en plusieurs positions discrètes préétablies, définies par les maillons, et non d'une possibilité de réglage continue le long des chaînes,
- selon les constatations des inventeurs, la concentration des différents capteurs peut encore être améliorée.

On connaît du document US 2014/0276183 A1 un autre dispositif pour la mesure de signaux de l'activité cérébrale destiné à être apposé sur la tête d'un individu.

La présente invention a pour but de pallier les inconvénients précités en proposant un dispositif pour la mesure de signaux d'activité cérébrale dont la structure autorise, au moins selon des modes de réalisation particuliers:
- l'accès au cuir chevelu et de manière à dégager les cheveux des capteurs en particulier des émetteurs/détecteurs d'optodes, et/ou des électrodes,
- un confort de portage pour l'utilisateur, sans création de pression trop importante à l'origine de douleurs,
- un réglage des positions des différents supports capteur(s), en différentes positions, et de préférence de manière continue le long de guides latéraux,
- de procéder au montage du dispositif, y compris d'effectuer des pré-réglages des positions des différents supports de capteur(s) sur une tête de mannequin, puis de retirer le dispositif ainsi préparé et afin de l'apposer sur la tête d'un individu, avantageusement sans perte des réglages de positionnement,
- de porter un nombre important de capteurs, en particulier des électrodes et des optodes, et selon une densité spatiale permettant l'acquisition simultanée de signaux par électroencéphalographie et par spectroscopie dans le proche infrarouge, sur des zones d'intérêt ciblées.

Aussi l'invention concerne un dispositif convenant pour la mesure des signaux de l'activité cérébrale d'un individu, destiné à être apposé sur la tête dudit individu comportant une structure destinée au port de capteurs, ladite structure autorisant un

Selon l'invention, ladite structure du dispositif comporte :
- un support central, de préférence déformable, apte à s'adapter à la courbure de tête, destiné à être positionné le long de la tête, de préférence suivant le plan médian du crâne,
- des supports latéraux, s'étendant latéralement audit support central, espacés les uns des autres,
- des supports de capteur(s), solidaires et fixés auxdits supports latéraux, de positions réglables le long des guides souples,
- un système de mise en tension desdits supports latéraux.

Selon l'invention lesdits supports latéraux sont des guides souples, le long desquels lesdits supports de capteur(s) sont de positions réglables.

Selon l'invention, lesdits supports de capteur(s) sont de positions réglables le long des guides souples, lesdits supports de capteur(s) présentant deux passants pour les guides souples autorisant un tel réglage de position, lesdits supports de capteurs comportant chacun une partie de support présentant :
- un pied, en particulier élastomère, destiné à appuyer sur la tête du porteur, dans une position centrale par rapport aux positions des différents capteurs supportés, les deux dits passants étant positionnés sur le support de capteur, respectivement de part et d'autre dudit pied,
- une surface dorsale sur laquelle appuie la section de longueur dudit guide souple intermédiaire entre les deux passants, ladite surface dorsale étant positionnée juste au-dessus du pied, afin d'augmenter la stabilité du support de capteurs.

Selon l'invention, tout ou partie des supports de capteur(s) sont des supports de capteurs dits multiples, permettant chacun le support d'au moins deux capteurs physiquement distincts ;

Selon des caractéristiques optionnelles de l'invention prises seules ou en combinaison :
- ledit système de mise en tension desdits guides souples, comporte, d'une part, de chaque côté de la tête du porteur, une armature, rigide, destinée à s'étendre au moins localement sous l'oreille, les deux armatures étant de préférence reliées par une mentonnière, et d'autre part, des élastiques, chacun solidaire par l'une de ses extrémités à l'extrémité terminale inférieure de l'un correspondant desdits guides souples, chaque élastique étant destiné à être fixé à ladite armature pour assurer la mise en tension du guide souple correspondant ;
- ledit système de mise en tension comporte un moyen de réglage de la tension de l'élastique ;
- le moyen de réglage de la tension de l'élastique comporte une ouverture de passage de l'armature pour l'élastique, ladite ouverture de passage étant dimensionnée inférieure par rapport à la section de l'élastique, et de telle sorte que :
   - un effort manuel de traction sur l'extrémité libre de l'élastique permet le glissement de l'élastique au travers de ladite ouverture de passage, et ainsi un réglage par augmentation de la tension, et lorsque l'effort manuel n'est plus,
   - les frottements entre l'ouverture de passage et l'élastique sont tels qu'ils interdisent le glissement, assurant la mise en tension du guide souple correspondant ;
- les guides souples sont dans un matériau inélastique ;
- les guides souples sont dans un textile ;
- lesdits guides souples sont des bandes ;
- une fois l'opération manuelle de réglage effectuée, le maintien en position du support de capteurs sur le guide souple est assuré par les frottements entre le guide souple et le support de capteurs ;
- lesdits supports de capteurs, dits multiples, comprennent des supports de capteurs pour deux capteurs, destinés à être positionnés de part et d'autre dudit pied d'appui ;
- lesdits supports de capteurs, dit multiples, comprennent des supports de capteurs pour quatre capteurs, destinés à être répartis autour dudit pied d'appui ;
- chaque capteur est destiné à être monté mobile en coulissement par rapport à la partie du support de capteur équipé du pied d'appui, un élément ressort, tel qu'un ressort de compression, étant configuré pour contraindre le capteur en direction vers et en appui sur la tête de l'individu ;
- ladite partie du support de capteur(s) équipée dudit pied d'appui étant nommée partie fixe, ledit support de capteur(s) comprend, pour le ou chaque capteur, une partie mobile par rapport à ladite partie fixe, sur laquelle est solidarisé le capteur, ledit élément ressort étant prévu entre ladite partie fixe et ladite partie mobile dudit support de capteur(s) ;
- ladite partie fixe comporte, pour le ou chaque capteur, une conformation creuse, en particulier cylindrique, à l'intérieur de laquelle est destinée à glisser ladite partie mobile et dans lequel ladite partie mobile du support de capteur(s) présente une extrémité terminale pour la fixation d'un capteur, ainsi qu'une surface de guidage destinée à coulisser le long de la paroi intérieure de la conformation creuse ;
- ladite partie mobile présente une partie saillante s'étendant au travers d'une ouverture supérieure de la conformation creuse, un organe de verrouillage, tel qu'une goupille, étant monté de manière amovible sur la partie saillante, de manière à, d'une part, assurer le maintien et le verrouillage de ladite partie mobile à ladite partie fixe dudit support de capteur(s) et d'autre part, permettre, une fois l'organe de verrouillage retiré, le retrait et le démontage de la partie mobile par rapport à ladite partie fixe ;
- l'organe de verrouillage prend la forme d'une boucle, destinée à la préhension, et permettant d'écarter le capteur de la surface de la tête lorsqu'un effort de traction est effectué sur la boucle ;
- ledit support central présente, suivant le sens de la longueur, une pluralité de passages transversaux, mutuellement espacés suivant la longueur dudit support central pour le positionnement et le maintien respectif d'une pluralité de guides souples, suivant différentes positions d'écartement définies entre lesdits guides souples ;
- ledit support central, forme suivant le sens de la longueur, un canal longitudinal, à fonction de guide-câbles, destiné pour le guidage des liaisons filaires des différents capteurs ;
- le dispositif comprend un boitier recevant une électronique d'acquisition des signaux des capteurs ;
- le dispositif présente des capteurs comportant des électrodes destinées à la mesure de signaux par électroencéphalographie et/ou des détecteurs et émetteurs optiques pour la mesure de signaux par spectroscopie dans le proche infrarouge ;
- le dispositif comprend une coiffe amovible, en un matériau occultant, destinée à être appliquée sur la structure support de capteurs du dispositif, et de manière à isoler les capteurs des interférences lumineuses de l'environnement ambiant.

L'invention concerne encore l'utilisation d'un dispositif conforme à l'invention équipé des capteurs comportant lesdites électrodes destinées à la mesure de signaux par électroencéphalographie et lesdits détecteurs et émetteurs optiques pour la mesure de signaux par spectroscopie dans le proche infrarouge, pour la mesure simultanée desdits signaux par électroencéphalographie et par spectroscopie dans le proche infrarouge.

L'invention sera mieux comprise à la lecture de la description suivante accompagnée des dessins en annexe parmi lesquels :
- La figure 1 est une vue en perspective d'un dispositif conforme à l'invention, appliqué sur la tête d'un individu,
- Les figures 2, 3 et 4 sont respectivement des vues en perspective, de dessus, une vue de coupe selon la coupe III-III de la figure 3, d'un support de capteurs, à deux capteurs,
- La figure 5 est une vue de coupe de la partie mobile du support de capteurs, conforme à l'invention selon un mode de réalisation, et convenant à un type de capteur, en particulier une électrode,
- La figure 6a est une vue de coupe de la partie mobile du support de capteurs, conforme à l'invention selon un mode de réalisation, et convenant à un type de capteur, en particulier un émetteur d'une optode,
- La figure 6b est une vue de coupe de la partie mobile du support de capteurs, conforme à l'invention selon un mode de réalisation, et convenant à un type de capteur, en particulier un détecteur d'une optode,
- Les figures 7, 8 et 9 sont respectivement des vues en perspective, de dessus, et de coupe selon la coupe VIII-VIII de la figure 8, d'un support de capteurs à quatre capteurs,

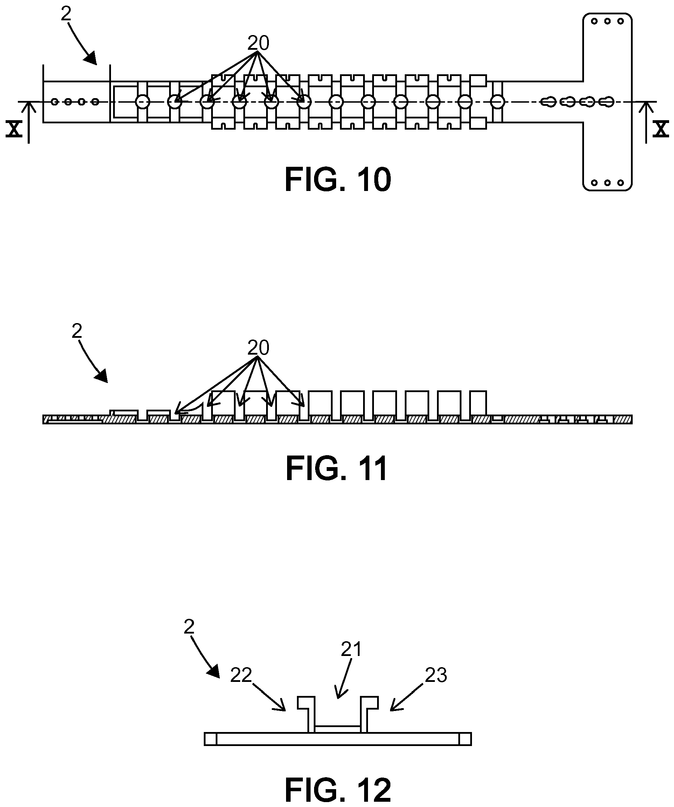

- Les figures 10, 11 et 12, sont respectivement des vues de dessus, de coupe selon la coupe X-X de la figure 10, et de face, d'un support central, selon un exemple,
- Les figures 13 et 14 sont des vues de côté et de face de l'armature (droite) du dispositif,
- La figure 15 est une vue éclatée d'un boitier, de son capot, ainsi que d'une carte électronique pour acquisition des signaux de mesure.

Aussi, l'invention concerne un dispositif 1 convenant pour la mesure des signaux de l'activité cérébrale d'un individu, destiné à être apposé sur la tête dudit individu comportant une structure de support destinée au port de capteurs, ladite structure autorisant un réglage de la position des capteurs.

Selon l'invention, ladite structure du dispositif comporte :
- un support central 2, de préférence déformable, apte à s'adapter à la courbure de tête, destiné à être positionné le long de la tête, de préférence suivant le plan médian du crâne,
- des guides souples 3, s'étendant latéralement audit support central, en particulier de part et d'autre dudit support central, espacés les uns des autres,
- des supports de capteur(s) 4₁, 4₂, fixés et solidaires auxdits guides souples, de positions réglables le long des guides,
- un système 5, 6 de mise en tension desdits guides souples 3.

Les guides souples 3 sont répartis sur la surface de la tête, mutuellement espacés les uns des autres, selon des écartements prédéfinis, en particulier par ledit support central 2. Cette structure permet, lors de la pose, l'accès au cuir chevelu, et ainsi de dégager les cheveux des capteurs positionnés par les supports de capteurs au niveau des différents inter-espaces entre les guides souples.

Les supports de capteur(s) 4₁, 4₂ sont fixés et solidaires auxdits guides souples 3, mais toutefois de positions réglables le long de ces guides. Cette possibilité de réglage de position permet notamment d'adapter les positions des différents capteurs en fonction des zones d'intérêts à étudier.

Le fait que les support de capteur(s) 4₁, 4₂ soient solidaires des guides souples 3, et non seulement en simple appui, permet avantageusement d'effectuer un pré-montage de la structure, y compris un préréglage des positions des différents capteurs sur une tête de mannequin, puis de retirer et d'apposer la structure sur la tête de l'individu, et avantageusement sans perte des réglages de position le long des guides souples 3.

Ledit système de mise en tension desdits guides souples peut comporter, d'une part, de chaque côté de la tête du porteur, une armature 6, rigide ou semi rigide, destinée à s'étendre au moins localement sous l'oreille, les deux armatures 6 étant de préférence reliées par une mentonnière 61, et d'autre part, des élastiques 5, chacun solidaire par l'une de ses extrémités à l'extrémité terminale inférieure de l'un correspondant desdits guides souples 3, chaque élastique 5 étant destiné à être fixé à ladite armature 6 pour assurer la mise en tension du guide souple 3 correspondant.

On notera que, selon ce mode de réalisation avantageux, les guides souples 3 et les élastiques 5 correspondants sont des éléments distincts : il est ainsi possible de choisir pour lesdits guides souples un matériau inélastique (par exemple un textile classique) par comparaison au matériau des élastiques 5 qui peuvent être en silicone ou dans un autre matériau du type élastomère.

Selon les constatations des inventeurs, le choix d'un matériau inélastique pour les guides souples 3 (par comparaison au matériau des élastiques 5), permet avantageusement de garantir, lors d'un préréglage de la structure sur une tête de mannequin, que les capteurs seront, une fois la structure retirée du mannequin et apposer sur la tête dudit individu, positionnés sensiblement en les mêmes positions, et sans décalage de positionnement tels qu'il seraient rencontrés dans le cas où les guides souples seraient eux-mêmes dans le même matériau que les élastiques 5.

En particulier, et dans le cas où les supports de capteurs 4₁ ; 4₂ sont fixés aux guides souples grâce à des passants 40,41 ; 42,44 ; 43,45 des supports, traversés par les guides souples 3, et tels que décrits par la suite, le fait de choisir les guides souples en un matériau inélastique (ou inextensible) permet d'éviter, ou à tout le moins de limiter, les phénomènes de réduction de section des guides souples lorsque ces derniers sont mis en tension, réduction de section qui, dans le cas où les guides souples seraient en un matériau élastique, provoquerait une perte de position des supports de capteurs le long des guides souples.

Selon un mode de réalisation, les guides souples 3 et les supports de capteurs 4₁, 4₂ sont agencés et tels que les guides souples 3 sont en appui sur la tête dudit individu, en tout ou partie, au niveau des sections de longueur du guide souple 3, intermédiaires, entre les supports de capteurs 4₁,4₂. Afin de permettre la stabilité de l'appui du guide souple 3 et une répartition homogène, chaque guide souple peut prendre la forme d'une bande, destinée à venir en appui sur la tête, par l'une de ses faces.

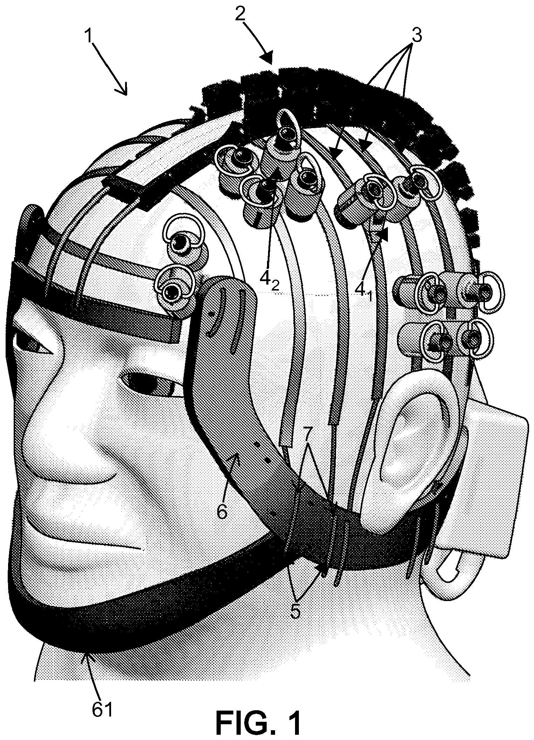

Selon un mode de réalisation ledit système de mise en tension comporte un moyen de réglage de la tension de l'élastique. Par exemple, et tel qu'illustré non limitativement à la figure 1, le moyen de réglage de la tension de l'élastique (de chaque élastique 5) comporte une ouverture de passage 7 de l'armature pour l'élastique 5, ladite ouverture de passage 7 étant dimensionnée inférieure par rapport à la section de l'élastique 5, et de telle sorte que :
- un effort manuel de traction sur l'extrémité libre de l'élastique 5 permet le glissement de l'élastique au travers de ladite ouverture de passage 7, et ainsi un réglage par augmentation de la tension, et lorsque l'effort manuel n'est plus,
- les frottements entre l'ouverture de passage 7 et l'élastique 5 sont tels qu'ils interdisent le glissement, assurant la mise en tension du guide souple correspondant.

Un tel système de mise en tension permet aussi un réglage de la tension de chaque guide souple 3, par la mise en tension dudit élastique correspondant, permettant ainsi de contraindre sur la tête l'ensemble des supports de capteurs porté par ce guide souple. Il s'agit avantageusement d'une possibilité de réglage continu. D'autres modes de réalisation de moyen de réglage de la tension sont possibles, en particulier, des réglages du type discontinu, par exemple en prévoyant plusieurs positions d'accroche de l'élastique (non illustré) sur l'armature et afin de permettre différents efforts de tension.

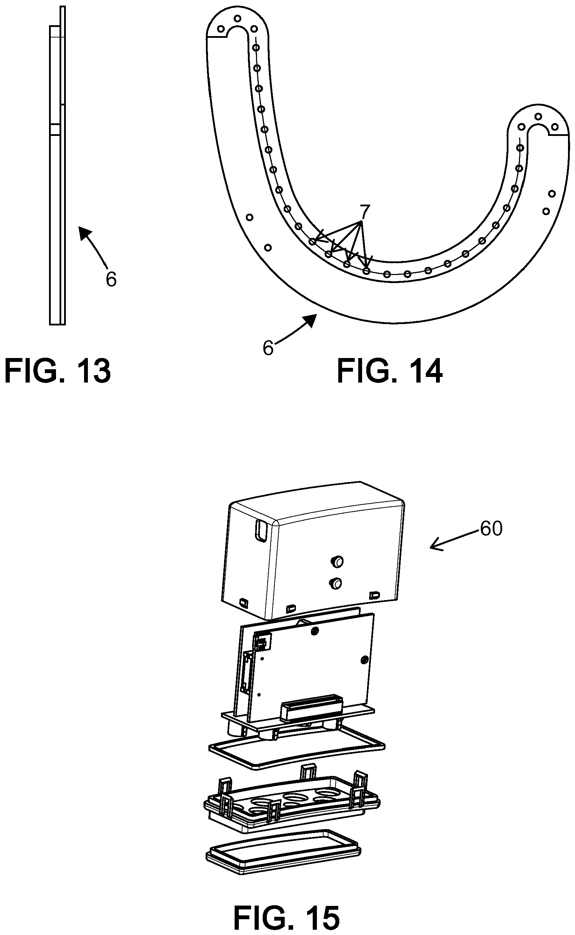

L'armature peut être un élément en plastique de forme courbe, tel qu'illustré aux figures 13 et 14, destiné à s'étendre, par exemple, depuis la partie arrière de la tête, en passant sous l'oreille et jusqu'à une zone latérale à proximité du front. Sur sa longueur, une pluralité d'ouvertures de passage 7 peut permettre l'insertion des élastiques 5 en les positions les plus adaptées au réglage. Sur la face intérieure en appui avec la peau, cet élément peut être recouvert d'une matière souple.

La possibilité de réglage des supports de capteur(s) 41, 42 le long des guides peut être assurée par des passants 40, 41 ; 42, 43, 44, 45 pour les guides souples autorisant un tel réglage de position. Le réglage de position s'effectue avantageusement, sans retirer le support de capteur(s) de son guide souple, en le faisant glisser manuellement le long du guide souple 3. Une fois cette opération manuelle exécutée, c'est-à-dire la position dudit support de capteur(s) réglée, le maintien en position du support de capteurs sur le guide souple 3 est assuré avantageusement (uniquement) par les frottements entre le guide souple et le support de capteurs 41,42.

Selon un mode de réalisation, lesdits supports de capteurs 41,42 comportent chacun une partie présentant un pied 46 destiné à appuyer sur la tête du porteur. Dans le cas d'un support de capteurs multiple (supportant plusieurs capteurs physiquement distincts), ce pied 46, d'appui est de préférence en une position centrale par rapport aux positions des différents capteurs supportés. Ce pied peut être avantageusement en une matière souple, différente du corps du support, par exemple en un élastomère.

Selon un mode de réalisation, ladite partie dudit support de capteurs présentant le pied 46 d'appui peut comprendre deux dits passants 40,41 ; 42,44 ; 43,45 , positionnés sur le support de capteur, respectivement de part et d'autre dudit pied 46, tous deux traversés par l'un des guides souples 3, ainsi qu'une surface dorsale 47 sur laquelle appuie la section de longueur dudit guide souple intermédiaire entre les deux passants 40,41 ; 42,44 ; 43,45.

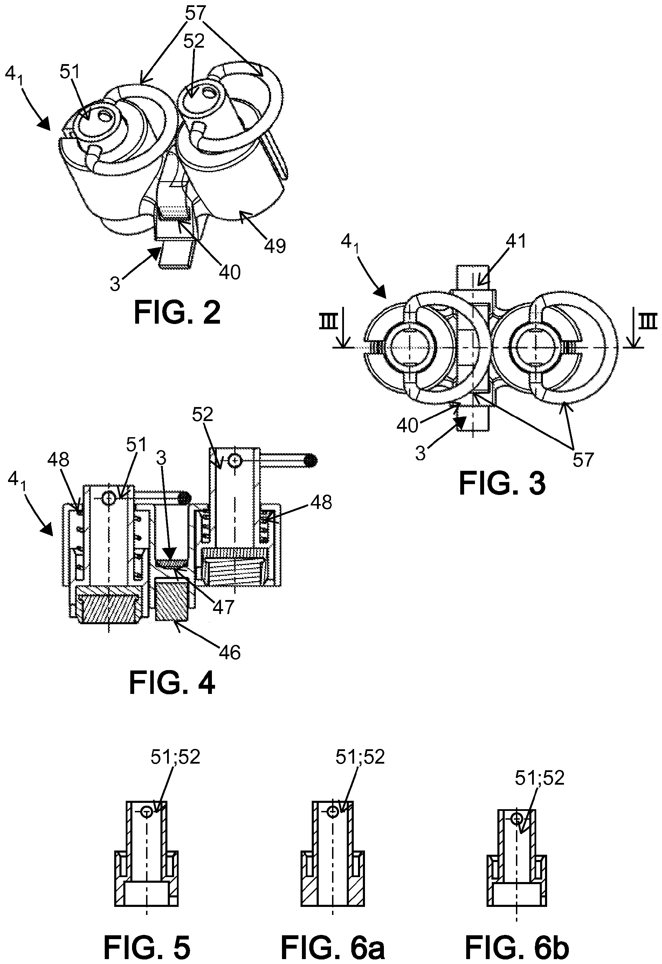

Selon ce mode de réalisation, illustré non limitativement, notamment aux figures 2 et 3, le guide souple 3, notamment la bande, traverse un premier passant 40, à partir du dessous du support 4₁, s'étend en appui le long de la surface dorsale 47, puis traverse un deuxième passant 41, à partir du dessus du support. On notera que la surface dorsale 47 peut être avantageusement positionnée juste au-dessus du pied 46, et afin d'augmenter la stabilité (au basculement) du support de capteurs 4₁,4₂.

Avantageusement tout ou partie des supports de capteur(s) peuvent être des supports de capteurs 4₁, 4₂, dit multiple, permettant chacun le support d'au moins deux capteurs physiquement distincts. La présence de tels supports de capteurs multiples permet avantageusement de densifier la position des capteurs sur une zone d'intérêt.

Lesdits supports de capteurs, dits multiples, peuvent comprendre des supports de capteurs 4₁ pour deux capteurs, destinés à être positionnés de part et d'autre dudit pied 46 d'appui. Un tel mode de réalisation est illustré à titre non limitatif des figures 2 à 4. Dans un tel cas, le pied 46 est positionné à mi-distance des capteurs, qui sont alors positionnés de part et d'autre du guide souple 3 sur lequel est solidaire ledit support de capteurs 41. De tels supports de capteurs (« double ») trouveront un intérêt particulier pour positionner des capteurs sur des zones du crâne présentant une forte courbure.

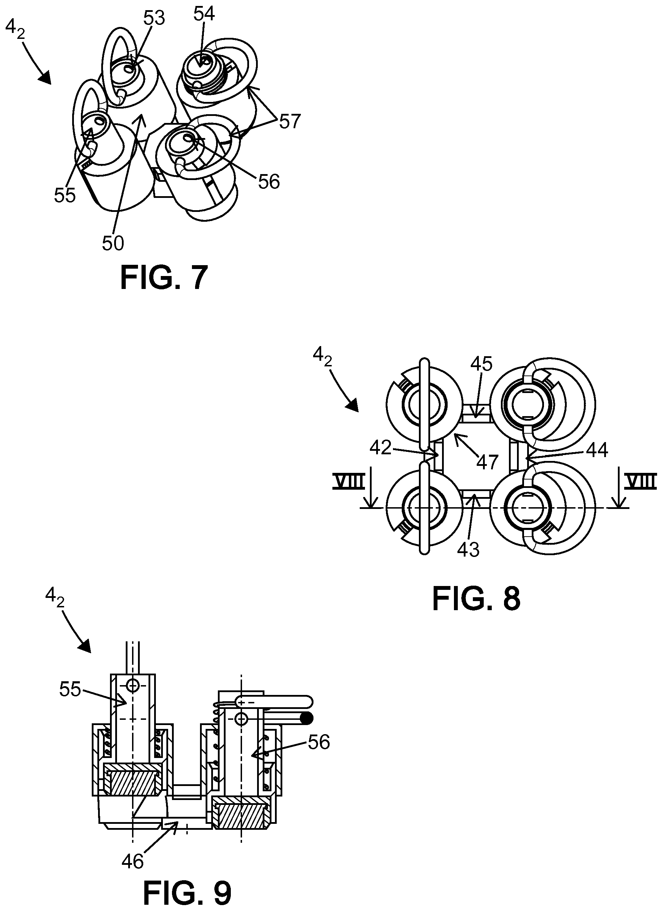

Les supports de capteurs, dit multiple, peuvent comprendre des supports de capteurs 4₂ pour quatre capteurs (« quadruple »), destinés à être répartis autour dudit pied d'appui 46. Les quatre capteurs peuvent être positionnés aux quatre sommets d'un rectangle (par exemple un carré). Un tel mode de réalisation est illustré à titre non limitatif des figures 7 à 8. Un tel mode de réalisation est particulièrement intéressant en ce qu'il permet de regrouper quatre capteurs, de manière rapprochée sur une même zone d'intérêt de crane, mais nécessite toutefois d'être appliqué sur une zone de crâne relativement plan.

Ainsi et tel qu'illustré à la figure 1, les supports de capteurs *(« double* »*)* et les supports de capteurs *(« quadruple* »*)* peuvent être combinés sur la structure. Il est bien entendu que la figure 1 est une figure partielle en ce qu'elle illustre seulement quelques supports de capteurs, les autres n'étant pas illustrés, dans un souci de clarté, afin de laisser voir la structure, et en particulier les différents guides souples 3.

Selon un mode de réalisation avantageux, chaque capteur est destiné à être monté mobile en coulissement par rapport à la partie du support de capteurs équipé du pied 46 d'appui, un élément ressort 48, tel qu'un ressort de compression, étant configuré pour contraindre le capteur en direction vers et en appui sur la tête du porteur.

Avantageusement, un tel mode de réalisation permet de s'assurer que le ou chaque capteur soit bien positionné en appui sur la tête de l'individu, tout en maitrisant l'effort de pression dudit capteur sur la tête.

A cet effet, l'élément ressort 48, notamment le ressort de compression est choisi de manière à ce que l'effort de rappel soit suffisant pour plaquer le capteur sur la tête, mais de raideur suffisamment faible pour que cet effort ne soit pas douloureux. Dans le cas d'un support de capteurs multiples, chaque capteur est mobile par rapport à la partie du support équipé du pied 46 et de manière indépendante par rapport aux autres capteurs. Chaque capteur présente un élément ressort 48, indépendant. Comme illustré non limitativement à la figure 4 (à droite), la course du capteur est telle, qu'en fin de course haute, il s'élève substantiellement au-dessus du niveau de l'appui du pied 46. Autrement dit, lorsque le capteur est au niveau correspondant à la surface d'appui du pied comme illustré non limitativement à la figure 4 (à gauche), ou au voisinage de cette dernière, l'effort du capteur sur la tête est limité par le choix de l'élément ressort 48, et en particulier le choix de sa raideur, et son taux de compression à cette position (la précontrainte). A titre d'exemple non limitatif, l'élément ressort 48 peut présenter une raideur de 40 N/m, la précontrainte définie à 0,25 N lorsque l'extrémité inférieure du capteur est à même niveau que la surface d'appui du pied 46. La structure du dispositif permet une répartition des charges diminuant les zones de pressions ponctiformes.

Selon un mode de réalisation, ladite partie du support de capteur(s) équipée dudit pied d'appui 46 étant nommée partie fixe 49; 50, ledit support de capteur(s) 4₁; 4₂ comprend, pour le ou chaque capteur, une partie mobile 51,52 ; 53, 54, 55, 56 par rapport à ladite partie fixe solidaire dudit capteur, ledit élément ressort 48 étant prévu entre ladite partie fixe et ladite partie mobile dudit support de capteurs.

A cet effet et selon un mode de réalisation illustré, ladite partie fixe 49 ; 50 comporte, pour le ou chaque capteur, une conformation creuse, en particulier cylindrique, à l'intérieur de laquelle est destinée à glisser ladite partie mobile. Ladite partie mobile du support de capteur(s) présente une extrémité terminale, inférieure pour la fixation d'un capteur, ainsi qu'une surface de guidage, notamment cylindrique, destinée à coulisser le long de la paroi intérieure de la conformation creuse.

Tel qu'illustré aux figures 5 et 6a et 6b notamment, la partie mobile 51 ou 52 du support de capteurs peut être spécifique à un type de capteur particulier, jouant ainsi le rôle d'adaptateur. Le dispositif peut ainsi comprendre un jeu de parties mobiles, constituant respectivement différents adaptateurs pour le montage de différents capteurs physiquement distincts tels que des électrodes, ou encore l'émetteur ou encore le détecteur d'une optode. Par exemple, la partie mobile du support illustré à la figure 5 est un adaptateur permettant la fixation d'une électrode, alors que les parties mobiles de la figure 6a et de la figure 6b permettent respectivement le montage de la partie émettrice et détectrice d'une optode.

Selon un mode de réalisation, ladite partie mobile présente un partie saillante s'étendant au travers d'une ouverture supérieure de la conformation creuse, un organe de verrouillage 57, tel qu'une goupille, étant monté de manière amovible sur la partie saillante, de manière à, d'une part, assurer le maintien et le verrouillage de ladite partie mobile 51,52 ; 53, 54, 55, 56 à ladite partie fixe 49; 50, dudit support de capteur(s) 4₁; 4₂, et d'autre part, permettre, une fois l'organe de verrouillage retiré, le retrait et le démontage de la partie mobile 51,52 ; 53, 54, 55, 56 par rapport à ladite partie fixe 49; 50. L'assemblage et le désassemblage de l'organe de verrouillage 57 sont de préférence effectués sans outil.

L'organe de verrouillage 57 peut avantageusement prendre la forme d'une boucle, destinée à la préhension, et permettant d'écarter le capteur de la surface de la tête lorsqu'un effort de traction est effectué sur la boucle. La boucle est un élément déformable élastiquement, par exemple métallique présentant deux extrémités libres destinées à venir s'insérer dans deux orifices correspondants de la partie saillante.

Tel qu'illustré à la figure 4, à gauche cet organe de verrouillage 57, une fois monté, vient en butée avec la partie fixe, en fin de course basse, interdisant le retrait de la partie mobile de la conformation creuse de la partie fixe. Le retrait de la boucle s'effectue en déformant la boucle et de manière à sortir les extrémités libres des orifices. Une fois la boucle retirée, la partie mobile peut être extraite de la conformation creuse.

Le corps de partie fixe du capteur peut être une pièce plastique, notamment moulé par injection, le pied constitué par un élément élastomère rapporté sur le corps. De même, chaque partie mobile 51,52 ; 53, 54, 55, 56 peut être un élément plastique, notamment moulé par injection.

Ledit support central 2 peut présenter, suivant le sens de la longueur, une pluralité de passages 20, transversaux, notamment sous la forme de rainures, mutuellement espacés suivant la longueur dudit support central pour les positionnements et les maintiens respectifs d'une pluralité de guides souples 3, suivant différentes positions d'écartement définies entre lesdits guides souples.

Ledit support central 2, peut présenter un canal longitudinal 2 suivant le sens de la longueur, à fonction de guide-câbles, destiné pour le guidage des liaisons filaires des différents capteurs. Le support central 2 peut être un élément en élastomère qui comprend une bande centrale, dont la surface inférieure est destinée à prendre appui sur la tête, et la surface supérieure est pourvue desdits passages transversaux, en sur-profondeur et sous la forme de rainures, respectivement réparties le long de la bande. Ledit support central peut présenter des bords relevés 22,23, de manière discontinue, entre les rainures, le long des deux bords longitudinaux de la bande et formant, avec ladite bande centrale, ledit canal longitudinal 21. Le support central 2 peut être en une matière élastomère, et notamment obtenu par moulage.

Ce canal 21 permet de guider les liaisons filaires des capteurs en direction d'un boitier recevant une électronique d'acquisition des signaux des capteurs, ledit boitier étant solidaire de la structure, positionné en partie arrière de la tête. Ce boitier reçoit intérieurement une électronique d'acquisition des signaux de mesure, en particulier des signaux par spectroscopie dans le proche infrarouge et/ou des signaux par électroencéphalographie. Dans le cas de la mise en œuvre d'une spectroscopie proche de l'infrarouge, le dispositif peut encore comprendre une coiffe (non illustrée), amovible, en un matériau occultant, destinée à être appliquée sur la structure support de capteurs du dispositif, et de manière à isoler les capteurs (en particulier les optodes) des interférences lumineuses de l'environnement ambiant. Une telle coiffe permet avantageusement au porteur de pouvoir procéder aux mesures NIRS, dans le respect des conditions d'enregistrement (c'est-à-dire dans l'obscurité), et sans nécessiter de plonger le porteur dans l'obscurité. La coiffe peut être dans un tissu occultant, léger et afin de ne pas appuyer sur les supports de capteur(s), et présenter un lien de serrage et/ou un élastique, sur son pourtour afin de pouvoir ajuster la coiffe à différentes tailles de tête.

### NOMENCLATURE

1. Dispositif,
2. Support central,
3. Guides souples,
4₁, 4₂. Supports de capteurs (respectivement à deux capteurs ou quatre capteurs),
5. Elastiques,
6. Armatures,
7. Ouvertures de passage (Armatures 6),

20. Passages transversaux (Support central 2),
21. Canal longitudinal (Support central 2),
22,23. Bords relevés (Support central 2).

40, 41. Passants (Supports capteurs 4₁),
42, 43, 44, 45. Passants (Supports capteurs 4₂),
46. Pied (Support de capteurs 4₁ ou 4₂),
47. Surface dorsale
48. Elément ressort (par exemple ressort de compression),
49. Partie fixe (Supports de capteurs 4₁),
50. Partie fixe (Supports de capteurs 4₂),
51,52. Parties mobiles (Supports de capteurs 4₁),
53, 54, 55, 56. Parties mobiles (Supports de capteurs 4₂),
57. Organe de verrouillage (notamment boucle de préhension),

60. Boitier (électronique d'acquisition),
61. Mentonnière.

## Revendications

1. Dispositif (1) convenant pour la mesure des signaux de l'activité cérébrale d'un individu, destiné à être apposé sur la tête dudit individu comportant une structure (2) destinée au port de capteurs, ladite structure autorisant un réglage de la position des capteurs,
dans lequel ladite structure du dispositif comporte :
- un support central (2), déformable, apte à s'adapter à la courbure de tête, destiné à être positionné le long de la tête, de préférence suivant le plan médian du crâne,
- des supports latéraux (3) s'étendant latéralement audit support central, espacés les uns des autres,
- des supports de capteur(s) (4₁, 4₂), solidaires et fixés auxdits support latéraux (3), tout ou partie des supports de capteur(s) (4₁, 4₂) étant des supports de capteurs (4₁, 4₂), dits multiples, permettant chacun le support d'au moins deux capteurs physiquement distincts,
- un système (5,6) de mise en tension desdits supports latéraux (3), où
lesdits supports latéraux (3) sont des guides souples (3), le long desquels lesdits supports de capteur(s) (4₁, 4₂) sont de positions réglables, **caractérisé en ce que**
lesdits supports de capteur(s) (4₁, 4₂) présentent deux passants (40, 41 ; 42, 44 ; 43, 45) pour les guides souples autorisant un tel réglage de position,
lesdits supports de capteurs comportant chacun une partie de support présentant :
- un pied (46), en particulier élastomère, destiné à appuyer sur la tête du porteur, dans une position centrale par rapport aux positions des différents capteurs supportés, les deux dits passants (40, 41 ;42, 44 ; 43, 45) étant positionnés sur le support de capteur, respectivement de part et d'autre dudit pied,
- une surface dorsale (47) sur laquelle appuie la section de longueur dudit guide souple intermédiaire entre les deux passants (40, 41 ;42, 44 ; 43, 45), ladite surface dorsale (47) étant positionnée juste au-dessus du pied (46), afin d'augmenter la stabilité du support de capteurs (4₁, 4₂)..

2. Dispositif selon la revendication 1, dans lequel ledit système de mise en tension desdits guides souples, comporte, d'une part, de chaque côté de la tête du porteur, une armature (6), rigide, destinée à s'étendre au moins localement sous l'oreille, les deux armatures étant de préférence reliées par une mentonnière, et d'autre part, des élastiques (5), chacun solidaire par l'une de ses extrémités à l'extrémité terminale inférieure de l'un correspondant desdits guides souples (3), chaque élastique (5) étant destiné à être fixé à ladite armature (6) pour assurer la mise en tension du guide souple (3) correspondant.

3. Dispositif selon la revendication 2, dans lequel ledit système de mise en tension comporte un moyen de réglage de la tension de l'élastique.

4. Dispositif selon la revendication 3, dans lequel le moyen de réglage de la tension de l'élastique comporte une ouverture de passage (7) de l'armature pour l'élastique (5), ladite ouverture de passage (7) étant dimensionnée inférieure par rapport à la section de l'élastique (5), et de telle sorte que :
- un effort manuel de traction sur l'extrémité libre de l'élastique (5) permet le glissement de l'élastique au travers de ladite ouverture de passage (7), et ainsi un réglage par augmentation de la tension, et lorsque l'effort manuel n'est plus,
- les frottements entre l'ouverture de passage (7) et l'élastique (5) sont tels qu'ils interdisent le glissement, assurant la mise en tension du guide souple correspondant.

5. Dispositif selon l'une des revendications 1 à 4 dans lequel les guides souples (3) sont dans un matériau inélastique.

6. Dispositif selon la revendication 5, dans lequel les guides souples (3) sont dans un textile.

7. Dispositif selon l'une des revendications 1 à 6, dans lequel lesdits guides souples (3) sont des bandes.

8. Dispositif selon la revendication 1, dans lequel, une fois l'opération manuelle de réglage effectuée, le maintien en position du support de capteurs sur le guide souple est assuré par les frottements entre le guide souple (3) et le support de capteurs (41,42).

9. Dispositif selon l'une des revendications 1 à 8, dans lequel lesdits supports de capteurs, dits multiples, comprennent des supports de capteurs (4₁) pour deux capteurs, destinés à être positionnés de part et d'autre dudit pied (46) d'appui.

10. Dispositif selon l'une des revendications 1 à 9, dans lequel lesdits supports de capteurs, dits multiples, comprennent des supports de capteurs (4₂) pour quatre capteurs, destinés à être répartis autour dudit pied d'appui (46).

11. Dispositif selon l'une des revendications 1 à 10, dans lequel chaque capteur est destiné à être monté mobile en coulissement par rapport à la partie du support de capteurs équipé du pied (46) d'appui, un élément ressort (48), tel qu'un ressort de compression, étant configuré pour contraindre le capteur en direction vers et en appui sur la tête du porteur.

12. Dispositif selon la revendication 11, dans lequel ladite partie du support de capteur(s) équipée dudit pied d'appui (46) étant nommée partie fixe (49; 50), ledit support de capteur(s) (4₁; 4₂) comprend, pour le ou chaque capteur, une partie mobile (51,52 ; 53, 54 ; 55, 56) par rapport à ladite partie fixe solidaire dudit capteur, ledit élément ressort (48) étant prévu entre ladite partie fixe et ladite partie mobile dudit support de capteur(s).

13. Dispositif selon la revendication 12, dans lequel ladite partie fixe (49 ;50) comporte, pour le ou chaque capteur, une conformation creuse, en particulier cylindrique, à l'intérieur de laquelle est destinée à glisser ladite partie mobile et dans lequel ladite partie mobile du support de capteur(s) présente une extrémité terminale pour la fixation d'un capteur, ainsi qu'une surface de guidage destinée à coulisser le long de la paroi intérieure de la conformation creuse.

14. Dispositif selon la revendication 13, dans lequel ladite partie mobile présente une partie saillante s'étendant au travers d'une ouverture supérieure de la conformation creuse, un organe de verrouillage (57), tel qu'une goupille, étant monté de manière amovible sur la partie saillante, de manière à, d'une part, assurer le maintien et le verrouillage de ladite partie mobile (51,52 ; 53, 54, 55, 56) à ladite partie fixe (49; 50), dudit support de capteur(s) (4₁; 4₂), et d'autre part, permettre, une fois l'organe de verrouillage retiré, le retrait et le démontage de la partie mobile (51,52 ; 53, 54, 55, 56) par rapport à ladite partie fixe (49; 50).

15. Dispositif selon la revendication 14, dans lequel l'organe de verrouillage (57) prend la forme d'une boucle, destinée à la préhension, et permettant d'écarter le capteur de la surface de la tête lorsqu'un effort de traction est effectué sur la boucle.

16. Dispositif selon l'une des revendications 1 à 15, dans lequel ledit support central (2) présente, suivant le sens de la longueur, une pluralité de passages (20), transversaux, mutuellement espacés suivant la longueur dudit support central pour le positionnement et le maintien respectifs d'une pluralité de guides souples (3), suivant différentes positions d'écartement définies entre lesdits guides souples.

17. Dispositif selon l'une des revendications 1 à 16, dans lequel ledit support central (2), forme suivant le sens de la longueur, un canal longitudinal (21), à fonction de guide-câbles, destiné pour le guidage des liaisons filaires des différents capteurs.

18. Dispositif selon l'une des revendications 1 à 17, comprenant un boitier recevant une électronique d'acquisition des signaux des capteurs.

19. Dispositif selon l'une des revendications 1 à 18, dans lequel les capteurs comportent des électrodes destinées à la mesure de signaux par électroencéphalographie et/ou des détecteurs et émetteurs optiques pour la mesure de signaux par spectroscopie dans le proche infrarouge.

20. Dispositif selon l'une des revendications 1 à 19 comprenant une coiffe amovible, en un matériau occultant, destinée à être appliquée sur la structure support de capteurs du dispositif, et de manière à isoler les capteurs des interférences lumineuses de l'environnement ambiant.

21. Utilisation d'un dispositif selon la revendication 19 équipé des capteurs comportant lesdites électrodes destinées à la mesure de signaux par électroencéphalographie et lesdits détecteurs et émetteurs optiques pour la mesure de signaux par spectroscopie dans le proche infrarouge, pour la mesure simultanée desdits signaux par électroencéphalographie et par spectroscopie dans le proche infrarouge.

## Patentansprüche

1. Vorrichtung (1), die zum Messen der Signale der Hirnaktivität eines Individuums geeignet ist, die dazu bestimmt ist, auf den Kopf des Individuums gesetzt zu werden, umfassend eine Struktur (2), die zum Tragen von Sensoren bestimmt ist, wobei die Struktur eine Einstellung der Position der Sensoren gestattet,
wobei die Struktur der Vorrichtung umfasst:
- einen zentralen Träger (2), der verformbar und geeignet ist, sich an die Krümmung eines Kopfes anzupassen, der dazu bestimmt ist, entlang des Kopfes, vorzugsweise entlang der Mittelebene des Schädels, positioniert zu sein,
- seitliche Träger (3), die sich seitlich zum zentralen Träger erstrecken und voneinander beabstandet sind,
- Sensorträger (4₁, 4₂), die mit den seitlichen Trägern (3) verbunden und an diesen befestigt sind, wobei die Gesamtheit oder ein Teil der Sensorträger (4₁, 4₂) so genannte Mehrfachsensorträger (4₁, 4₂) sind, die jeweils das Tragen mindestens zwei physisch getrennter Sensoren ermöglichen,
- ein System (5, 6) zum Spannen der seitlichen Träger (3), wobei die seitlichen Träger (3) flexible Führungen (3) sind, entlang derer die Sensorträger (4₁, 4₂) in ihrer Position einstellbar sind,
**dadurch gekennzeichnet, dass** die Sensorträger (4₁, 4₂) zwei Durchgänge (40, 41; 42, 44; 43, 45) für die flexiblen Führungen aufweisen, die eine solche Positionseinstellung gestatten,
wobei die Sensorträger jeweils einen Tragteil umfassen, umfassend:
- einen Fuß (46), insbesondere aus Elastomer, der dazu bestimmt ist, auf dem Kopf des Trägers in einer zentralen Position in Bezug zu den Positionen der verschiedenen getragenen Sensoren aufzuliegen, wobei die zwei Durchgänge (40, 41; 42, 44; 43, 45) auf dem Sensorträger jeweils beiderseits des Fußes positioniert sind,
- eine Rückseite (47), auf der der Längsabschnitt der flexiblen Zwischenführung zwischen den zwei Durchgängen (40, 41; 42, 44; 43, 45) aufliegt, wobei die Rückseite (47) direkt über dem Fuß (46) positioniert ist, um die Stabilität des Sensorträgers (4₁, 4₂) zu erhöhen.

2. Vorrichtung nach Anspruch 1, bei der das System zum Spannen der flexiblen Führungen einerseits auf jeder Seite des Kopfes des Trägers einen starren Beschlag (6), der dazu bestimmt ist, sich zumindest lokal unter dem Ohr zu erstrecken, wobei die zwei Beschläge vorzugsweise durch einen Kinnriemen verbunden sind, und andererseits Gummibänder (5) umfasst, die jeweils mit einem ihrer Enden mit dem unteren Ende der einen entsprechenden flexiblen Führung (3) verbunden sind, wobei jedes Gummiband (5) dazu bestimmt ist, an dem Beschlag (6) befestigt zu sein, um das Spannen der entsprechenden flexiblen Führung (3) zu gewährleisten.

3. Vorrichtung nach Anspruch 2, bei der das System zum Spannen ein Mittel zum Einstellen der Spannung des Gummibandes umfasst.

4. Vorrichtung nach Anspruch 3, bei der das Mittel zum Einstellen der Spannung des Gummibandes eine Durchgangsöffnung (7) des Beschlags für das Gummiband (5) umfasst, wobei die Durchgangsöffnung (7) kleiner als der Querschnitt des Gummibandes (5) dimensioniert ist, so dass:
- eine manuelle Zugkraft auf das freie Ende des Gummibandes (5) das Gleiten des Gummibandes durch die Durchgangsöffnung (7) und somit eine Einstellung durch Erhöhung der Spannung ermöglicht, und wenn die manuelle Kraft nicht mehr vorhanden ist,
- die Reibungen zwischen der Durchgangsöffnung (7) und dem Gummiband (5) derart sind, dass sie das Gleiten untersagen, was das Spannen der entsprechenden flexiblen Führung gewährleistet.

5. Vorrichtung nach einem der Ansprüche 1 bis 4, bei der die flexiblen Führungen (3) aus einem nicht elastischen Material sind.

6. Vorrichtung nach Anspruch 5, bei der die flexiblen Führungen (3) aus Stoff sind.

7. Vorrichtung nach einem der Ansprüche 1 bis 6, bei der die flexiblen Führungen (3) Bänder sind.

8. Vorrichtung nach Anspruch 1, bei der, wenn der manuelle Einstellvorgang durchgeführt ist, der Halt des Sensorträgers in Position auf der flexiblen Führung durch die Reibungen zwischen der flexiblen Führung (3) und dem Sensorträger (41, 42) gewährleistet ist.

9. Vorrichtung nach einem der Ansprüche 1 bis 8, bei der die so genannten Mehrfachsensorträger Sensorträger (4₁) für zwei Sensoren umfassen, die dazu bestimmt sind, beiderseits des Stützfußes (46) positioniert zu sein.

10. Vorrichtung nach einem der Ansprüche 1 bis 9, bei der die so genannten Mehrfachsensorträger Sensorträger (4₂) für vier Sensoren umfassen, die dazu bestimmt sind, um den Stützfuß (46) verteilt zu sein.

11. Vorrichtung nach einem der Ansprüche 1 bis 10, bei der jeder Sensor dazu bestimmt ist, gleitbeweglich in Bezug zu dem Teil des Sensorträgers montiert zu sein, der mit dem Stützfuß (46) ausgestattet ist, wobei ein Federelement (48), wie eine Kompressionsfeder, eingerichtet ist, um den Sensor in Richtung zu dem und auf den Kopf des Trägers zu zwingen.

12. Vorrichtung nach Anspruch 11, bei der der Teil des Sensorträgers, der mit dem Stützfuß (46) ausgestattet ist, fester Teil (49; 50) genannt wird, wobei der Sensorträger (4₁, 4₂) für den oder jeden Sensor einen beweglichen Teil (51, 52; 53, 54; 55, 56) in Bezug zu dem mit dem Sensor verbundenen festen Teil umfasst, wobei das Federelement (48) zwischen dem festen Teil und dem beweglichen Teil des Sensorträgers vorgesehen ist.

13. Vorrichtung nach Anspruch 12, bei der der feste Teil (49; 50) für den oder jeden Sensor eine hohle, insbesondere zylindrische, Ausbildung umfasst, in der der bewegliche Teil bestimmt ist zu gleiten, und in der der bewegliche Teil des Sensorträgers ein Ende für die Befestigung eines Sensors aufweist, sowie eine Führungsfläche, die dazu bestimmt ist, entlang der Innenwand der hohlen Ausbildung zu gleiten.

14. Vorrichtung nach Anspruch 13, bei der der bewegliche Teil einen vorspringenden Teil aufweist, der sich durch eine obere Öffnung der hohlen Ausbildung erstreckt, wobei ein Verriegelungselement (57), wie in Stift, abnehmbar auf dem vorspringenden Teil montiert ist, um einerseits den Halt und die Verriegelung des beweglichen Teils (51, 52; 53, 54, 55, 56) am festen Teil (49; 50) des Sensorträgers (4₁, 4₂) zu gewährleisten, und andererseits, wenn das Verriegelungselement herausgezogen ist, das Herausziehen und die Demontage des beweglichen Teils (51, 52; 53, 54, 55, 56) in Bezug zum festen Teil (49; 50) zu ermöglichen.

15. Vorrichtung nach Anspruch 14, bei der das Verriegelungselement (57) die Form einer Schleife annimmt, die zum Ergreifen bestimmt ist und es ermöglicht, den Sensor von der Oberfläche des Kopfes zu entfernen, wenn eine Zugkraft auf die Schleife ausgeübt wird.

16. Vorrichtung nach einem der Ansprüche 1 bis 15, bei der der zentrale Träger (2) entlang seiner Längsrichtung eine Vielzahl von Querdurchgängen (20) aufweist, die entlang der Länge des zentralen Trägers für die jeweilige Positionierung und den jeweiligen Halt einer Vielzahl von flexiblen Führungen (3) in verschiedenen Abstandspositionen, die zwischen den flexiblen Führungen definiert sind, beabstandet sind.

17. Vorrichtung nach einem der Ansprüche 1 bis 16, bei der der zentrale Träger (2) in seiner Längsrichtung einen Längskanal (21) mit Kabelführungsfunktion bildet, der für die Führung der Drahtverbindungen der verschiedenen Sensoren bestimmt ist.

18. Vorrichtung nach einem der Ansprüche 1 bis 17, umfassend ein Gehäuse, das eine Elektronik zur Erfassung der Signale der Sensoren aufnimmt.

19. Vorrichtung nach einem der Ansprüche 1 bis 18, bei der die Sensoren Elektroden, die zum Messen von Signalen durch Elektroenzephalografie bestimmt sind, und/oder optische Detektoren und Sender zum Messen von Signalen durch Nah-Infrarot-Spektroskopie umfassen.

20. Vorrichtung nach einem der Ansprüche 1 bis 19, umfassend eine abnehmbare Haube aus abschirmendem Material, die dazu bestimmt ist, auf die Sensortragstruktur der Vorrichtung aufgesetzt zu werden, um die Sensoren gegen Lichtinterferenzen der Umgebung zu isolieren.

21. Verwendung einer Vorrichtung nach Anspruch 19, die mit Sensoren ausgestattet ist, umfassend Elektroden, die zum Messen von Signalen durch Elektroenzephalografie bestimmt sind, und optische Detektoren und Sender zum Messen von Signalen durch Nah-Infrarot-Spektroskopie zum gleichzeitigen Messen der Signale durch Elektroenzephalografie und Nah-Infrarot-Spektroskopie.

## Claims

1. A device (1) suitable for measuring brain activity signals of an individual, intended to be placed on the head of said individual including a structure (2) intended for carrying sensors, said structure allowing for an adjustment of the position of the sensors,
wherein said device structure includes :
- a central support (2), which is deformable, able to adapt to the head curvature, intended to be positioned along the head, preferably following the median plane of the skull,
- lateral supports (3), extending laterally to said central support, spaced from each other,
- sensor supports (4₁, 4₂), which are integral and fixed to said lateral supports (3), all or part of the sensor supports (4₁, 4₂) being so-called multiple sensor supports (4₁, 4₂) each allowing for the support of at least two physically distinct sensors,
- a system (5, 6) for tensioning said lateral supports (3), where said lateral supports are flexible guides (3), along which the sensor supports (4₁, 4₂) have adjustable positions,
**characterised in that**
said sensor supports (4₁, 4₂) provide two loop holders (40, 41; 42, 44; 43, 45) for the flexible guides allowing for such position adjustment,
said sensor supports each including a support portion providing:
- a foot (46), in particular in elastomer form, intended to press against the wearer's head, in a central position with respect to the positions of the various supported sensors, said two loop holders (40, 41; 42, 44; 43, 45) being positioned on the sensor support, on either side of said foot, respectively,
- a back surface (47) on which the length section of said intermediate flexible guide between the two loop holders (40, 41; 42, 44; 43, 45) is resting, said back surface (47) being positionned just over the foot (46), in such a way as to increase the stability of the sensor support (4₁, 4₂).

2. The device according to Claim 1, wherein said system for tensioning said flexible guides includes, on the one hand, on each side of the wearer's head, a rigid armature (6) which is intended to extend at least locally under the ear, the two armatures preferably being connected by a chin strap, and on the other hand, rubber bands (5), which are each secured by one of its ends to the lower terminal end of a corresponding one of said flexible guides (3), each rubber band (5) being intended to be fixed to said armature (6) in order to ensure the tensioning of the corresponding flexible guide (3).

3. The device according to Claim 2, wherein said tensioning system includes means for adjusting the tension of the rubber band.

4. The device according to Claim 3, wherein the means for adjusting the tension of the rubber band include a passage opening (7) in the armature for the rubber band (5), said passage opening (7) being dimensioned to be smaller than the cross-section of the rubber band (5), and such that:
- a manual pulling force on the free end of the rubber band (5) allows the rubber band to slide through said passage opening (7), and thus allows an adjustment by increasing the tension, and when the manual effort is no longer,
- the friction between the passage opening (7) and the rubber band (5) is such that it will not allow any sliding, ensuring the tensioning of the corresponding flexible guide.

5. The device according to one of Claims 1 to 4 wherein the flexible guides (3) are made of an inelastic material.

6. The device according to Claim 5, wherein the flexible guides (3) are in a fabric.

7. The device according to one of Claims 1 to 6, wherein said flexible guides (3) are strips.

8. The device according to Claim 1, wherein, after the manual adjustment operation has been carried out, maintaining the sensor support in position on the flexible guide is ensured by the friction between the flexible guide (3) and the sensor support (4₁, 4₂).

9. The device according to one of Claims 1 to 8, wherein said so-called multiple sensor supports comprise sensor supports (4₁) for two sensors, intended to be positionned on either side of said supporting foot (46).

10. The device according to one of Claims 1 to 9, wherein said so-called multiple sensor supports comprise sensor supports (4₂) for four sensors, intended to be distributed around said supporting foot (46).

11. The device according to one of Claims 1 to 10, wherein each sensor is intended to be slidably mounted with respect to the part of the sensor support that is equipped with the supporting foot (46), a spring element (48), such as a compression spring, being configured so as to force the sensor in the direction towards and pressing against the head of the carrier.

12. The device according to Claim 11, wherein said part of the sensor support that is equipped with said supporting foot (46) being named fixed part (49; 50), said sensor support (4₁; 4₂) comprises, for the sensor or for each sensor, a part (51, 52; 53, 54; 55, 56) which is movable with respect to said fixed part that is integral with said sensor, said spring element (48) being provided between said fixed part and said movable part of said sensor support.

13. The device according to Claim 12, wherein said fixed part (49; 50) includes, for the sensor or for each sensor, a hollow, in particular cylindrical, conformation inside which said movable part is intended to slide and in which said movable part of the sensor support has a terminal end for fixing a sensor, as well as a guide surface intended to slide along the inner wall of the hollow conformation.

14. The device according to Claim 13, wherein said movable part has a protruding part extending through an upper opening of the hollow conformation, a locking member (57), such as a pin, being removably mounted on the protruding part, so as to, on the one hand, ensure the holding and locking of said movable part (51, 52; 53, 54, 55, 56) to said fixed part (49; 50) of said sensor support (4₁; 4₂), and on the other hand, allowing, once the locking member is removed, the removal and disassembling of the movable part (51, 52; 53, 54, 55, 56) with respect to said fixed part (49; 50).

15. The device according to Claim 14, wherein the locking member (57) takes the form of a loop, intended for gripping, and allowing the sensor to be separated from the surface of the head when a tensile force is applied to the loop.

16. The device according to one of Claims 1 to 15, wherein said central support (2) has, along the lengthwise direction, a plurality of crosswise passageways (20), which are mutually spaced apart along the length of said central support for the respective positioning and holding of a plurality of flexible guides (3), according to different spacing positions defined between said flexible guides.

17. The device according to one of Claims 1 to 16, wherein said central support (2) forms a longitudinal channel (21) along the lengthwise direction, with the function of a cable guide, intended for guiding the cable connections of the various sensors.

18. The device according to one of Claims 1 to 17, comprising a housing receiving electronics for acquiring sensor signals.

19. The device according to one of Claims 1 to 18, wherein the sensors include electrodes which are intended for the measurement of signals by electroencephalography and/or optical detectors and transmitters for the measurement of signals by near infrared spectroscopy.

20. The device according to one of Claims 1 to 19 comprising a removable upper cover, made of an occulting material, intended to be applied to the sensor support structure of the device, and in such a way as to isolate the sensors from light interference from the ambient environment.

21. A use of a device according to Claim 19 equipped with sensors comprising said electrodes intended for measuring signals by electroencephalography and said optical detectors and transmitters for measuring signals by near infrared spectroscopy, for simultaneous measurement of said signals by electroencephalography and near infrared spectroscopy.
